Europäisches Patentamt

European Patent Office

Office européen des brevets

(19)

(11) Numéro de publication : **0 457 950 B1**

(12) **FASCICULE DE BREVET EUROPEEN**

(45) Date de publication du fascicule du brevet :
**20.10.93 Bulletin 93/42**

(51) Int. Cl.⁵ : **A61K 31/20**

(21) Numéro de dépôt : **90109883.0**

(22) Date de dépôt : **23.05.90**

(54) **Utilisation de l'acide stéaridonique pour le traitement des maladies inflammatoires.**

(43) Date de publication de la demande :
**27.11.91 Bulletin 91/48**

(45) Mention de la délivrance du brevet :
**20.10.93 Bulletin 93/42**

(84) Etats contractants désignés :
**BE CH DE DK ES FR GB IT LI LU NL SE**

(56) Documents cités :
**EP-A- 0 068 854**
**EP-A- 0 087 865**
**EP-A- 0 295 954**
**EP-A- 0 302 482**
**EP-A- 0 347 056**
**GB-A- 2 204 239**
**Lipids, vol. 24, no. 12, déc. 1989, pp. 1004-1007;**
**V. Kockmann et al.**

(56) Documents cités :
**Int. Archs Allergy Appl. Immun., vol. 34, nov. 1987, pp. 233-240; S. Yoshino et al.**
**Prostaglandins, vol. 28, no. 5, nov. 1984, p. 668; T. Terano et al.**
**Allergie et Immunologie, vol. 19, no. 8, oct. 1987, pp. 12-13; J.L. Robin**
**J. Rhenmatol, vol. 16, 1989, pp. 729-733; G. Tate et al.**
**Prostaglandins 1974, 8: 509-519, Willis et al.**
**Prostaglandins 1973, 4: 863-875, Silver et al.**

(73) Titulaire : **SOCIETE DES PRODUITS NESTLE S.A.**
**Case postale 353**
**CH-1800 Vevey (CH)**

(72) Inventeur : **Guichardant, Michel**
**32, Bd de la Forêt**
**CH-1009 Pully (CH)**
Inventeur : **Rigaud, Michel**
**5, rue Bizet**
**F-87000 Limoges (FR)**

## Description

L'invention a trait à l'utilisation de l'acide stéaridonique pour la fabrication d'une composition pharmaceutique anti-inflammatoire.

L'acide stéaridonique (SA, C18:4 Δ 6,9,12,15) est un acide gras polyinsaturé, de la série n-3 (nomenclature définie par l'emplacement de la première double liaison à partir du groupe méthyl). Pour être utilisé par l'organisme, l'acide alpha-linolénique (ALA, C 18:3 Δ 9,12,15), qui constitue le point de départ de la série n-3 et qui est présent dans la plupart des diètes ingérées, doit être métabolisé en SA par désaturation au moyen de l'enzyme Δ 6 désaturase. Or on sait que l'activité de cette enzyme est faible et qu'elle diminue lors du vieillissement et à la suite de certaines maladies.

Parmi les substances jouant un rôle important dans les processus inflammatoires, par exemple de type allergiques tels que l'asthme, de type cutané comme l'acné, le psoriasis et l'eczéma, ou de type rhumatismal, ainsi que ceux consécutifs aux traumatismes, états de choc ou pathologies comme par exemple la mucoviscidose, les leucotriènes sont formés principalement par l'oxydation par les lipoxygénases de l'acide arachidonique (AA, C20:4 Δ 5,8,11,14) libéré à partir des membranes cellulaires. On sait en particulier que les leucocytes polymorphonucléés humains transforment l'AA en leucotriènes, particulièrement en acide (5S,12R)-5,12-dihydroxy-6,14-cis-8,10-trans-eicosatétraénoïque (LTB4) de forme stable par l'intermédiaire de la 5-lipoxygénase. Le LTB4 joue un rôle prédominant dans différents processus inflammatoires.

Pour cette raison, on tente de diminuer les facteurs de l'inflammation en développant des inhibiteurs de la 5-lipoxygénase. On a montré que certains acides gras supérieurs de la série n-3, par exemple selon Lee, T.H. et al, (1984) dans J. Clin. Invest 74, 1922-1933, que l'acide eicosapentaénoïque (EPA, C20:5 Δ 5,8,11,14,17) pouvait moduler la synthèse des leucotriènes et améliorer les états inflammatoires. Ceci a été également montré pour un acide gras de la série n-6, l'acide dihomogamma-linolénique (DHLA, C20:3 Δ 8,11,14), par exemple selon Tate, G. et al., (1989) J. Rheumatol. 16,729-733. Nous avons trouvé que le SA a la propriété d'inhiber la biosynthèse des leucotriènes.

EP-A-0 347 056 concerne l'utilisation de certains acides gras essentiels dont, entre autres, l'acide stéaridonique en combinaison avec des doses élevées et thérapeutiques de vitamine E pour la fabrication d'un médicament pour le traitement de désordres inflammatoires.

L'invention concerne donc l'utilisation de l'acide stéaridonique ou de l'un de ses dérivés pharmaceutiquement acceptable pour la préparation de compositions pharmaceutiques comportant au moins un acide gras essentiel destinées à la prévention et au traitement chez les mammifères des maladies d'origine inflammatoire, notamment les maladies allergiques, cutanées, rhumatismales et celles consécutives aux traumatismes, états de choc et pathologies, impliquant une inhibition de la 5-lipoxygénase dans l'oxydation de l'acide arachidonique à l'exception des compositions comportants 3% ou plus en poids de vitamine E par rapport aux acides gras essentiels.

Par dérivé pharmaceutiquement acceptable, on entend un sel, ester, ou amide correspondant à l'acide gras. Comme sel on préfère les sels d'acides aminés, par exemple d'arginine. Les esters préférés envisagés sont par exemple les mono-, di- ou triglycérides et les phospholipides.

Dans un mode de réalisation particulier, on utilise le SA en combinaison avec un ou des acide(s) gras polyinsaturé(s) inhibiteur(s) du métabolisme oxygéné de l'AA. Ce peut être un acide gras polyinsaturé inhibiteur de la 5-lipoxygénase de la série n-3, par exemple l'EPA, l'acide docosahexaénoïque (DHA, C22:6 Δ 4,7,10,13,16,19) ou de la série n-6, par exemple le DHLA ou son précurseur l'acide gamma-linolénique (GLA, C18:3 Δ 6,9,12), ou encore un dérivé pharmaceutiquement acceptable d 'un tel acide gras tel que défini ci-dessus. Dans une mode de réalisation préféré de cette utilisation combinée, on met en oeuvre un concentrat contenant essentiellement le GLA et le SA provenant de l'huile de pépins de cassis, par exemple préparé selon l'exemple 1.2 de la demande de brevet CH 1919/89-9 (EP-A-0 399 417).

Dans un autre mode de réalisation préféré de cette utilisation combinée, on met en oeuvre un concentrat contenant essentiellement le SA, l'EPA et le DHA provenant de l'huile de poisson, par exemple préparé selon le 1er par. de l'exemple 3 de la demande de brevet CH 1919/89-9 (EP-A-0 399 417).

Le SA ou le mélange d'acides gras polyinsaturés ou leurs dérivés utilisés peuvent être avantageusement protégés de l'oxydation par un antioxydant, par exemple le palmitate d'ascorbyle, les tocophérols, l'acide ascorbique en présence de lécithine ou un mélange de tels antioxydants.

Une composition pharmaceutique précédente peut se présenter sous différentes formes adaptées au mode d'administration, par exemple par voie orale, entérale, rectale, parentérale ou topique.

On peut par exemple préparer des capsules, gélules, suppositoires ou sirops.

Dans le cas d'une administration entérale ou parentérale, la composition se présente sous forme de solutions ou émulsions stabilisées chimiquement et physiquement, apyrogènes et stériles.

Dans le cas particulier d'une application topique, par exemple pour combattre les inflammations dermatologiques du type acné, eczéma ou psoriasis, la composition peut se présenter par exemple sous forme de pommade, onguent, crème ou lotion.

La dose administrée dépend du type et du degré de la maladie à traiter. Elle peut être de 0,05 à 10 g d'acide stéaridonique par jour en prise unique ou de préférence en 2 à 3 prises séparées.

Les exemples ci-après illustrent l'invention. Dans ces exemples, les parties et pourcentages sont pondéraux sauf indication contraire.

## Exemple 1

### Action du SA sur la biosynthèse des leucotriènes

Pour étudier l'action du SA sur la biosynthèse des leucotriènes, on incube des leucocytes humains isolés avec le SA et, à titre de comparaison avec l'EPA et le DHLA, on identifie les métabolites résultant de l'action de la 5-lipoxygénase sur l'AA par chromatographie bidimentionnelle sur couche mince (TLC) et on les analyse quantitativement par chromatographie liquide haute performance (HPLC).

Pour suivre les métabolismes de AA et SA, certaines expériences ont été faites avec ces acides gras marqués au C14 en position C1.

### 1. Conditions expérimentales

#### 1.1 Préparation des suspensions de cellules

On recueille du sang sur un mélange d'acide citrique, de citrate trisodique et de dextrose comme agent anti-coagulant à partir de donneurs normaux n'ayant pas pris de médicament pendant au moins 10 jours avant la prise de sang. Après centrifugation à 200 g pendant 15 min, on élimine le plasma riche en plaquettes et on recueille la fraction sanguine restante à laquelle on ajoute 0,5 volume d'une solution aqueuse contenant 3% de dextrane 250 et 0,9% de NaCl. On laisse les globules rouges sédimenter en environ 90 min à la température ambiante, puis on recueille le surnageant et on le centrifuge pendant 15 min à 80 g. On remet en suspension pendant 15 s ce sédiment obtenu correspondant à environ $2 \times 10^6$ cellules dans 1 ml d'eau distillée, puis on rétablit l'iso-osmolarité en ajoutant 1 ml de solution Tyrode/HEPES de concentration double. On centrifuge alors la suspension pendant 10 min. à 60 g, on recueille un sédiment de leucocytes, que l'on remet en suspension dans une solution Tyrode/HEPES contenant 2 mM (mmol/l) de calcium.

Après avoir stimulé les leucocytes par du calcium ionophore A23187 (1μM) de l'AA (20 μM) pendant 10 min. à 37°C, on les incube avec les acides gras (le cas échéant marqué au C14 en position 1 pour SA ou AA) dans les quantités indiquées ci-après au par. 2. pendant 30 min. à 37°C. On arrête l'incubation au moyen d'éthanol contenant 2 nanomoles d'acide 13-hydroxy-octadécadiénoïque (13-HODE) (obtenu par action de la lipoxygénase de soja sur l'acide linoléique) et 2 nanomoles de prostaglandine B2 (PGB2), à raison de 3 fois le volume d'éthanol par rapport au volume de la suspension.

On extrait les Lipides par le chloroforme à raison de 6 fois le volume de la suspension (sans l'éthanol).13-HODE et PGB2 sont utilisés comme standards pour l'analyse quantitative.

#### 1.2 Analyse des acides gras mono-hydroxylés

On sépare les lipides extraits sur des plaques de silicagel G par chromatographie bidimentionnelle sur couche mince. On développe les acides gras monohydroxylés dans la première dimension au moyen d'un mélange solvant hexane/diéthyl-éther/acide acétique glacial en proportion volumique 59/40/1, les acides gras di-hydroxylés restant à l'origine de la plaque. On détecte, le cas échéant, les acides gras mono-hydroxylés marqués avec un détecteur de radioactivité, on les récolte de la plaque ainsi que le 13-HODE, on les extrait avec le diéthyl-éther, on évapore le solvant sous azote, puis on les sépare par HPLC en phase inverse avec un mélange solvant d'élution méthanol/acide acétique aqueux de pH3 en proportion volumique 73/27. On les détecte par spectroscopie UV à la longueur d'onde 234 nm et on les quantifie par comparaison avec le standard 13-HODE en faisant l'hypothèse qu'ils ont tous la même extinction spécifique $3 \times 10^4$ $M^{-1}cm^{-1}$.

#### 1.3. Analyse des acides gras di-hydroxylés

On développe les acides gras di-hydroxylés restant à l'origine de la plaque dans la seconde dimension perpendiculairement à la première à l'aide d'un mélange solvant hexane/diéthyl-éther/acide acétique glacial en proportion volumique 25/74/1. Leur détection, récolte, extraction, séparation et analyse quantitative s'effectue comme indiqué en 1.2 ci-dessus, sauf que le mélange solvant d'élution dans l'HPLC est méthanol/acide acétique aqueux de pH3 en proportion volumique 66/34. On utilise PGB2 comme standard d'évaluation quantitative des acides gras di-hydroxylés selon leur absorbance UV maximale respective (longueur d'onde 270 nm, extinction $2,8 \times 10^4$ et $5 \times 10^4$ $M^{-1}$ $cm^{-1}$ pour $PGB_2$ et respectivement di-hydroxy dérivés).

## 2. Résultats

L'acide arachidonique (AA) (Sigma Chemical Company, St.Louis MO, USA) est utilisé comme contrôle, c'est-à-dire qu'il n'y a pas d'incubation des leucocytes avec d'autres acides gras.

Les acides gras incubés avec les leucocytes sont: l'acide stéaridonique (SA) obtenu à partir de l'huile de pépins de cassis, sous forme purifiée selon la demande de brevet CH 1919/89-9 (EP-A-0 399 417) l'acide eicosapentaénoïque (EPA) (Sigma Chemical Company) et l'acide di-homogammalinolénique (DHLA) (Sigma Chemical Company). l'acide 5-hydroxyeicosatétraénoïque (5-HETE) (Cayman Chemical, Ann Arbor Mi, USA), longueur d'onde maximum ($\lambda$ max):234 nm, temps de rétention (Rt):42,69 min, l'acide 5S,12S[E,Z,E,Z]-di-hydroxyeicosatétraénoïque (di-HETE) (M. Guichardant et al, biochem. J.256,879-883, 1988), $\lambda$ max:259, 268 et 279 nm, Rt: 29,96, l'acide 5S,12 R [Z,E,E,Z,]-5,12-hihydroxy-6,14-cis-8,10-trans-eicosatétraénoïque (LTB4) (Cayman Chemical), $\lambda$ max:261, 271 et 282 mm, Rt: 27,46 min, l'isomère A de LTB4, 5S,12R [E,E,E,Z]-LTB4 (M.Guichardant et ai, biochem. s.256,879-883,1988), Rt:21,46 et

l'isomère B de LTB4, 5S,12 S[E,E,E,Z]-LTB4 (M.Guichardant et ai, biochem. s.256,879-883,1988), Rt:24,27.

Les quantités de métabolites obtenues sont exprimées en picomole/$10^8$ leucocytes et représentent la moyenne de 5 expériences avec n déterminations. Les degrés de signification, P, sont déterminés par un test t par rapport au contrôle.

Les résultats sont indiqués dans le tableau ci-après:

## TABLEAU

| Acide gras incubé avec les leucocytes, stimulés par AA | Quantité d'acide gras incubé, µM | n | Quantité de métabolites, picomole / $10^8$ leucocytes | | | | |
|---|---|---|---|---|---|---|---|
| | | | 5-HETE | LTB4 | LTB4 isomère A | LTB4 isomère B | DI-HETE |
| SA | 10 | 4 | 8880 — | 10220 P<0,02 | 2670 -- | 2780 -- | 700 -- |
| SA | 20 | 6 | 7030 P<0,05 | 8480 P<0,01 | 2070 P<0,01 | 2200 P<0,05 | 510 -- |
| EPA | 20 | 4 | 5920 P<0,01 | 9090 P<0,01 | 2180 P<0,05 | 2730 -- | 510 -- |
| DHLA | 20 | 3 | 5350 P<0,01 | 3140 P<0,01 | 770 P<0,02 | 780 P<0,05 | 158 P<0,01 |
| Contrôle | 0 | 6 | 9080 | 17770 | 4080 | 4190 | 720 |

Légende: --: non significatif

Les résultats précédents montrent clairement que le SA, acide gras polyinsaturé de la série n-3 a un effet sur le métabolisme de l'AA conduisant aux leucotriènes par l'intermédiaire de la 5-lipoxygénase des leucocytes humains. Il diminue la formation des 5-HETE et DI-HETE d'environ 25% et celle des leucotriènes B4 d'environ 50%, c'est-à-dire de manière comparable à l'EPA qui est également un acide gras polyinsaturé de la série n-3 à concentration égale (20 μM).

Son activité est cependant moins forte que celle de l'acide di-homogammalinolénique, acide gras polyinsaturé de la série n-6 qui inhibe la formation des leucotriènes B4 à environ 80% et celle du 5-HETE à environ 40% à la même concentration (20 μM).

Son effet pourrait aussi être dû à certains de ses métabolites hydroxylés qui ont été détectés à partir du SA marqué.

Exemple 2-5

2. On prépare des capsules de gélatine pour l'administration orale contenant 250 mg de SA obtenu à partir de l'huile de pépins de cassis.

3. On prépare des capsules de gélatine pour l'administration orale contenant 250 mg de SA obtenu à partir de l'huile de poisson.

4. On prépare des capsules de gélatine pour l'administration orale contenant 500 mg d'un mélange d'acides gras contenant 80% de GLA et 15% de SA obtenu à partir de l'huile de pépins de cassis.

5. On prépare des capsules de gélatine pour l'administration orale contenant 500 mg d'un mélange d'acides gras contenant 16% de SA, 35% d'EPA et 39% de DHA à partir de l'huile de poisson.

Exemples 6-9

On prépare les compositions suivantes destinées à une application topique:

| 6. Onguent hydrophobe | % |
|---|---|
| SA | 5 |
| Polyéthylène micronisé | 10 |
| Myristate d'isopropyle | 85 |

### 7. Pommade hydrophobe     %

| | |
|---|---|
| SA | 1 |
| Triglycérides des acides caprique, caprylique et stéarique | 40 |
| Triglycérides des acides caprique et caprylique | 30 |
| Vaseline | 20 |
| Huile de vaseline | 9 |

### 8. Crème     %

| | |
|---|---|
| SA | 0,5 |
| Alcool cétylique | 6 |
| Alcool cétylique oxyéthyléné à 20 moles d'oxyde d'éthylène monostéarate de glycérol | |
| Triglycérides des acides caprique et caprylique | 15 |
| Propylèneglycol | 10 |
| Eau | 68,5 |

### 9. Lotion     %

| | |
|---|---|
| SA | 0,1 |
| Ethanol | 50 |
| Propylèneglycol | 49,9 |

Les compositions des exemples 6 à 9 précédents sont fabriquées et entreposées en atmosphère inerte et à l'abri de la lumière.

**Revendications**

1. Utilisation de l'acide stéaridonique ou de l'un de ses dérivés pharmaceutiquement acceptable pour la préparation de compositions pharmaceutiques comportant au moins un acide gras essentiel destinées à la prévention et au traitement chez les mammifères des maladies d'origine inflammatoire impliquant une inhibition de la 5-lipoxygénase dans l'oxydation de l'acide arachidonique à l'exception des compositions comportant 3% ou plus en poids de vitamine E par rapport aux acides gras essentiel.

2. Utilisation selon la revendication 1 dans laquelle l'origine inflammatoire implique une inhibition de la biosynthèse des leucotriènes et des acides hydroxyéicosatétraénoïques.

3. Utilisation selon les revendications 1 ou 2, dans laquelle la composition contient en outre un ou des acides gras polyinsaturé(s) inhibiteur(s) du métabolisme oxygéné de l'acide arachidonique ou un des dérivés(s) pharmaceutiquement acceptable(s) d'un ou de tel(s) acide(s).

4. Utilisation selon la revendication 3, dans laquelle la composition contient en outre l'acide éicosapentaénoïque et/ou l'acide docosahexaénoïque.

5. Utilisation selon la revendication 3, dans laquelle la composition contient en outre l'acide gammalinolénique et/ou l'acide dihomogammalinolénique.

6. Utilisation selon la revendication 3, sous forme d'un concentrat provenant de l'enrichissement de l'huile de pépins de cassis ou de l'huile de poisson.

7. Utilisation selon l'une des revendications 1 à 6 sous une forme adaptée à l'administration par voie orale, rectale, entérale ou parentérale.

8. Utilisation selon l'une des revendications 1 à 6 sous une forme adaptée à l'administration topique.

9. Utilisation selon la revendication 1 sous une forme fournissant une dose quotidienne de 0,05 à 10 g d'acide stéaridonique ou de l'un de ses dérivés pharmaceutiquement acceptable.

## Claims

1. The use of stearidonic acid or any of its pharmaceutically acceptable derivatives for the preparation of pharmaceutical compositions containing at least one essential fatty acid intended for the prevention and treatment of disorders of inflammatory origin in mammals involving the inhibition of 5-lipoxygenase in the oxidation of arachidonic acid except for compositions containing 3% by weight or more of vitamin E, based on the essential fatty acids.

2. The use claimed in claim 1, in which the inflammatory origin involves inhibition of the diosynthesis of leucotrienes and hydroxyeicosatetraenoic acids.

3. The use claimed in claim 1 or 2, characterized in that the composition additionally contains one or more polyunsaturated fatty acid(s) which inhibit the oxygenated metabolism of arachidonic acid or one or more pharmaceutically acceptable derivative(s) of one or more such acid(s).

4. The use claimed in claim 3, in which the composition additionally contains eicosapentaenoic and/or docosahexaenoic acid.

5. The use claimed in claim 3, in which the composition additionally contains γ-linolenic and/or dihomo-γ-linolenic acid.

6. The use claimed in claim 3 in the form of a concentrate emanating from the enrichment of black currant seed oil or fish oil.

7. The use claimed in any of claims 1 to 6 in a form suitable for oral, rectal, enteral or parenteral administration.

8. The use claimed in any of claims 1 to 6 in a form suitable for topical administration.

9. The use claimed in claim 1 in a form providing a daily dose of 0.05 to 10 g stearidonic acid or any of its pharmaceutically acceptable derivatives.

## Patentansprüche

1. Verwendung von Stearidonsäure oder eines ihrer pharmazeutisch akzeptablen Derivate zur Herstellung von pharmazeutischen Zusammensetzungen, die wenigstens eine essentielle Fettsäure enthalten, zur Vorbeugung oder zur Behandlung von Krankheiten mit entzündlichem Ursprung, die mit einer Inhibierung der 5-Lipoxygenase bei der Oxidation von Arachidonsäure verbunden sind, bei Säugetieren, ausgenommen Zusammensetzungen, die 3 Gew.-% oder mehr Vitamin E, bezogen auf die essentiellen Fettsäuren, enthalten.

2. Verwendung nach Anspruch 1, bei dem der entzündliche Ursprung eine Inhibierung der Biosynthese der Leuokotriene und der Hydroxyeicosatetraensäuren impliziert.

3. Verwendung nach den Ansprüchen 1 oder 2, bei der die Zusammensetzung außerdem entweder eine oder mehrere mehrfach ungesättigte Fettsäure(n) mit Inhibitorwirkung für den Sauerstoffstoffwechsel der Arachidonsäure oder eines oder mehrere pharmazeutisch akzeptable Derivate davon enthält.

4. Verwendung nach Anspruch 3, bei der die Zusammensetzung außerdem Eicosapentaensäure und/oder Docosahexaensäure enthält.

5. Verwendung nach Anspruch 3, bei der die Zusammensetzung außerdem gamma-Linolensäure und/oder Dihomogammalinolensäure enthält.

6. Verwendung nach Anspruch 3 in Form eines Konzentrats, das durch Anreicherung aus Öl aus Kernen der schwarzen Johannisbeere oder aus Fischöl gewonnen wurde.

7. Verwendung nach einem der Ansprüche 1 bis 6 in einer Form, die für eine orale, rektale, enterale oder parenterale Verabreichung geeignet ist.

8. Verwendung nach einem der Ansprüche 1 bis 6 in einer Form, die für eine topische Anwendung geeignet ist.

9. Verwendung nach Anspruch 1 in einer Form, die eine tägliche Dosis von 0,05 bis 10 g Stearidonsäure oder eines ihrer pharmazeutisch akzeptablen Derivate zur Verfügung stellt.